# EUROPEAN PATENT SPECIFICATION

(11) **EP 2 801 350 B1**
(45) Date of publication and mention of the grant of the patent: **01.08.2018**
(21) Application number: 14167322.8
(22) Date of filing: 07.05.2014
(51) Int. Cl.: A61K 9/28, A61K 31/165, A61K 9/20

(54) **Pharmaceutical Formulations of Lacosamide**
Pharmazeutische Formulierungen von Lacosamid
Formulations pharmaceutiques de lacosamide

(30) Priority: 08.05.2013 TR 201305490
(43) Date of publication of application: 12.11.2014
(73) Proprietor: Sanovel Ilac Sanayi ve Ticaret A.S., 34460 Istanbul (TR)
(72) Inventor: Türkyilmaz, Ali, 34460 Istanbul (TR); Turp, Ali Hasan, 34460 Istanbul (TR); Kirat Uzunogullari, Nur, 34460 Istanbul (TR); Gülkok, Yildiz, 34460 Istanbul (TR); Eceoglu, Melike, 34460 Istanbul (TR)
(74) Representative: Sevinç, Erkan

(56) References cited:
- WO-A1-2011/055385
- WO-A2-2011/101863
- WO-A2-2012/072256

## Description

### Field of Invention

The present invention relates to a dry granulation method for preparing a pharmaceutical formulation in the form of a tablet, comprising a therapeutically effective amount of lacosamide or a pharmaceutically acceptable salt, sodium stearyl fumarate and at least one excipient.

### Background of Invention

Lacosamide is an aminoacid derivative with an anticonvulsant activity useful in the adjunctive treatment of partial-onset seizures with or without secondary generalization in adults with epilepsy. It is also called as erlosamide or harkoseride. Its chemical name is (2R)-2-(Acetylamino)-3-methoxy-N-(phenylmethyl) propanamide and its chemical structure is shown in the Formula I.

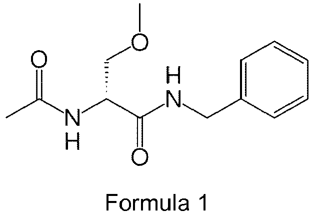

The precise mechanism of antiepileptic effects of lacosamide in humans remains to be fully elucidated. In vitro electrophysiological studies have shown that lacosamide selectively enhances slow inactivation of voltage-gated sodium channels, resulting in stabilization of hyperexcitable neuronal membranes and inhibition of repetitive neuronal firing. Inactivation of sodium channels is important to control of abnormal neuronal activity in the brain of epilepsy patient, and it can occur via fast or slow mechanism. While traditional sodium channel blocking antiepileptics (e.g., Lamotrigine, Carbamazepine, Oxcarbazepine or Phenytoin) act primarly via fast inactivation, lacosamide acts by selectively enhancing slow inactivation of voltage-gated sodium channels to control and stabilise the neural network in the brain.

Lacosamide is sparingly soluble in water and it has low flowability, which makes the tableting process difficult at the high amount of active substance in tablets.

There is lacosamide immediate release formulation licensed in the US and Europe in the form of immediate release tablets, oral solutions and intravenous injection solutions under the brand name Vimpat® by UCB. Vimpat® IR tablet has crospovidone as disintegrant agent, microcrystalline cellulose, HPC (low substituted) and HPC as filler and binder, silicified microcrystalline cellulose as glidant, colloidal silicon dioxide as filler, magnesium stearate as lubricant and a non-functional coating. It may be taken with or without food. The initial dose should be 50 mg twice daily (100 mg per day). Vimpat® IR tablet is prepared by wet granulation. To overcome poor flowability of lacosamide, wet granulation comprising HPC (including low substituted HPC) is selected.

Lacosamide can be increased at weekly intervals by 100 mg/day given as two divided doses up to the recommended maintenance dose of 200 to 400 mg/day, based on individual patient response and tolerability. The currently commercially available tablets are available in strengths of 50, 100, 150 and 200 mg of lacosamide.

WO 2012/072256 (A1) addresses the need for lacosamide formulations having improved side effect/efficacy ratio and benefit/risk ratio; and accordingly proposes lacosamide formulations having modified release profile. WO 2012/072256 (A1) does not suggest any solution for eliminating flowability, compressibility, disintegration, hardness and moisture related problems. Dry granulation method which comprises the use of sodium stearyl fumarate for preparing lacosamide tablet formulations is not known from WO 2012/072256 (A1).

WO2011/055385 (A1) relates to modified release lacosamide formulations. Modified release polymers such as hydrophilic polymer, hydrophobic polymer, wax are employed in lacosamide formulations. WO2011/055385 (A1) addressed the need for modified release formulation of lacosamide which controls the release of lacosamide in such a manner that therapeutically effective concentration is maintained in the blood for an extended period of time keeping the drug concentration in the blood substantially constant. Flowability, compressibility, disintegration, hardness and moisture related issues are not mentioned within WO2011/055385 (A1). Further, WO2011/055385 (A1) is also silent on the use of dry granulation method for preparing lacosamide tablet formulations in which sodium stearyl fumarate is employed.

PCT application WO 2010/060624 A2 Ratiopharm GMBH, 27.11.2008, provides preparation of lacosamide containing tablets wherein the tablets produced by means of dry granulation and direct compression to overcome poor flowability of lacosamide.

In this present invention, the solution to the flowability problem is producing granules that contain sodium stearyl fumarate as a hydrophilic lubricant instead of magnesium stearate which has some known drawbacks by means of dry granulation.

In addition, croscarmellose is used as a super-disintegrant which is not previously used in prior art to further improve the process.

### Description of the invention

The present invention provides a dry granulation method for preparing pharmaceutical tablet formulations comprising lacosamide or a pharmaceutically acceptable salt, sodium stearyl fumarate and at least one excipient to eliminate process problems as flowability and ensure rapid disintegration for desired drug dissolution characteristics.

The present invention provides a dry granulation method for preparing pharmaceutical tablet formulations comprising lacosamide or a pharmaceutically acceptable salt, sodium stearyl fumarate and at least one excipient to ensure a rapid disintegration and desired drug dissolution characteristic at the high dose (200mg or more) level of lacosamide that has flowability-problem.

In one aspect, the advantages of the formulations producing by dry granulation are well known by the skilled person in the art and such techniques were already studied for the lacosamide. However, the major difficulties of the development on dry granulation process are compressibility and flowability. Most of the directly compressible materials can accommodate only 30-40 % of the poorly compressible active ingredients which brings compressibility and also flowability problems.

The object of this invention is to produce dry granules comprising at least one hydrophilic lubricant to overcome compressibility and also flowability problems.

According to another embodiment of the present invention is to provide a dry granulation method for preparing a pharmaceutical formulation in the form of a tablet, comprising a therapeutically effective amount of lacosamide or a pharmaceutically acceptable salt, sodium stearyl fumarate and at least one excipient.

According to another object of the present invention is to provide a pharmaceutical formulation wherein lacosamide or a pharmaceutically acceptable salt thereof is present in an amount of about 5 to 80 % by weight of total composition, preferably it is about 20 to 50 % by weight of total composition.

In one aspect, the present invention relates to a lacosamide containing pharmaceutical composition does not comprise magnesium stearate. Magnesium stearate is a metal derivative lubricant. It has anti-adhesive and flow enhancement properties by ensuring uniformity of tablets. Besides this advantageous, its hydrophobic properties increase the process time with prolonged mixing. Because of this increase in time, lubricant forms a hydrophobic film around the active agent and retards the dissolution by retarding wetting of active agent.

According to one embodiment, the pharmaceutical formulation comprises at least one hydrophilic lubricant which is selected from the group comprising sodium stearyl fumarate, polyethylene glycol, sodium benzoate or a proper mixture thereof.

Accordingly, said pharmaceutical formulation does not comprise magnesium stearate.

In this invention, sodium stearyl fumarate is selected as a suitable lubricant in tableting. Due to hydrophilic property of sodium stearyl fumarate, formulation does not have the disadvantages of magnesium stearate in respect of tablet strength, disintegration and dissolution.

The amount of sodium stearyl fumarate is present in an amount of 0.01 to 10 % by weight of total composition; preferably it is 0.1 to 5% by weight of total composition.
In the present invention, said hydrophilic lubricant is sodium stearyl fumarate to overcome compressibility and flowability problem. The ratio of lacosamide to sodium stearyl fumarate is in the range of is in the range of 10 to 60 (w/w), preferably 20 to 30(w/w).

In this present invention, flowability test is performed by Erweka Gt Powder Tester instrument with cone diameter in 10 mm with a sample of 150 mL. The results shows that average angle of repose is between 41 to 45 degree.

In another aspect, the pharmaceutical formulation further comprises super- disintegrant to further improve the disintegration and dissolution of formulation.

As used herein, the term "super-disintegrant" is defined as the pharmaceutical ingredient that provides improved disintegration and dissolution. According to this embodiment, the term "super-disintegrant" is defined as croscarmellose sodium, sodium carboxymethyl starch, sodium starch glycolate, soy polysaccharide, cross-linked alginic acid, gellan gum, xanthan gum, calcium silicate or ion exchange resins preferably croscarmellose sodium.

According to one embodiment of the present invention, croscarmellose is used as a super-disintegrant which is not used in prior art.

According to this embodiment, the present invention relates to a dry granulation method for preparing a pharmaceutical formulation in the form of a tablet, comprising e therapeutically effective amount of lacosamide, sodium stearyl fumarate and croscarmellose sodium; wherein croscarmellose sodium is in the range of 0.1 - 10%, preferably 2 - 7%, and the ratio of lacosamide to croscarmellose sodium is in the range of 5 to 50(w/w), preferably 2 to 10(w/w).
The advantage of the formulation with crosscarmellose is to further improve disintegration and dissolution lacosamide by increasing the effect of sodium stearyl fumarate without using magnesium stearate that cause undesirable dissolution profile.

In another embodiment the ratio of croscarmellose sodium to sodium stearyl fumarate is in the range of preferably 1 to 10(w/w) and more preferably 2 to 5(w/w).

Suitable glidants may include but not limited to colloidal silicon dioxide, talc, aluminium silicate and the like and mixtures thereof, preferably colloidal silicon dioxide.

Suitable binders may include but not limited to polyvinylpyrrolidone, polyethylene glycol, polyvinyl alcohol, polyvinyl acetate and their copolymers, cellulose derivatives such as hydroxypropyl methyl cellulose, carboxy methyl cellulose, methyl cellulose, microcrystalline cellulose, gelatin, polyvinylalcohol, carrageenan, guar gum and the like and mixtures thereof; preferably polyvinylpyrrolidone - polyvinyl acetate copolymers.

Coating material may include but not limited to hydroxypropyl methyl cellulose (HPMC), polyethyleneglycol (PEG), polivinylpyrrolidon (PVP), vinylpyrrolidone-vinyl acetate copolymer (PVP- PVAc) and polivinyl alcohol and all OpadryTM derivatives and pigments, titanium dioxide, dyes and iron oxide and talc or mixtures thereof.

According to another embodiment, the formulation wherein minimum 85% of lacosamide by weight relative to the total weight of said formulation is released within 15 minutes when measured in 900mL of 0.1N HCl at 50 rpm named as USP Apparatus II (Paddle).

The pharmaceutical formulation consists of;
a) about 5 to 60% by weight of lacosamide
b) about 0.1 to 10% by weight of croscarmellose sodium
c) about 2 to 15% by weight of crospovidone
d) about 0.25 to 5% by weight polyvinylpyrrolidone - polyvinyl acetate copolymer
e) about 0.01 to 1% by weight colloidal silicon dioxide
f) about 10 to 60% by weight microcrystalline cellulose
g) about 0.1 to 5% by weight sodium stearyl fumarate
h) about 0.4 to 5% by weight coating

The pharmaceutical formulation is processed by means of dry granulation

According to another embodiment, the dry granulation method for preparing a pharmaceutical formulation of lacosamide, comprising the steps of
a) sieving lacosamide, 50% of sodium stearyl fumarate, 50% of crospovidone, 50% of colloidal siilicon dioxide and all other excipients and mixing the resulting mixture,
b) passing the mixture through a compactor or pressing into a slug briquette.
c) powders or slug briquetted tablets are crushed and sieved.
d) adding 50% of sodium stearyl fumarate, 50% of crospovidone, 50% of colloidal siilicon dioxide and mixing the resulting mixture,
e) pressing resultant mixture,

According to another embodiment, the dry granulation methodis carried out in a roller compactor and the rolling force is 180 to 190 kN

### Example 1: immediate release tablet

| **Ingredients** | **% amount (mg)** |
|---|---|
| lacosamide | 40 % |
| croscarmellose sodium | 5.0 % |
| crospovidone | 8.0 % |
| kollidon VA 64 | 2.0 % |
| colloidal silicon dioxide | 0.4 % |
| microcrystalline Cellulose (AviceIPh 112) | 43.1 % |
| sodium stearyl fumarate | 1.5 % |
| Opadry 200F (coating) | 3.0 % |

The production of the formulation is carried out as follows: 50% of sodium stearyl fumarate and all excipients other than colloidal silicon dioxide and lacosamide are weighed and pre-mixed for 10 min. Mixed powder passed through the compactor or pressed in slug briquettes. Powders or slug briquetted tablets are crushed and sieved. Colloidal silicon dioxide and other part of sodium stearyl fumarate are added and mixed for 5 minutes. Then, they are pressed and the tablets are coated preferably with OPADRY 200F.
For this example the average angle of repose has been found to be 44.2 degree.

With this invention, the dry granulation method for preparing a pharmaceutical tablet formulation comprising lacosamide or a pharmaceutically acceptable salt thereof eliminates any flowability and process related problems and bringing additional advantages. Method described above both serves both improved dissolution and disintegration and eliminates the need of magnesium stearate.

## Claims

1. A method for preparing a pharmaceutical formulation in the form of a tablet, comprising a therapeutically effective amount of lacosamide or a pharmaceutically acceptable salt, sodium stearyl fumarate and at least one excipient; wherein the method is dry granulation.

2. The method according to claim 1; wherein the amount of sodium stearyl fumarate is present in an amount of 0.01 to 10% by weight of total formulation; preferably it is 0.1 to 5% by weight of total formulation.

3. The method according to claims 1 and 2; wherein the ratio of lacosamide to sodium stearyl fumarate is in the range of 1 to 100 (w/w), preferably 20 to 30 (w/w).

4. The method according to claim 1; wherein the formulation further comprises a super - disintegrant.

5. The method according to claim 4; wherein said super - disintegrant is selected from the group comprising croscarmellose sodium, sodium carboxymethyl starch, sodium starch glycolate, soy polysaccharide, cross-linked alginic acid, gellan gum, xanthan gum, calcium silicate or ion exchange resins or mixtures thereof.

6. The method according to claim 5; wherein said super - disintegrant is croscarmellose sodium.

7. The method according to any of the preceding claims; wherein croscarmellose sodium is in the range of 0.1 - 10%, preferably 2 - 7% by weight of the total formulation.

8. The method according to any of the preceding claims; wherein the ratio of lacosamide to croscarmellose sodium is in the range of 5 to 50 (w/w), preferably 2 to 10 (w/w).

9. The method according to any of the preceding claims; wherein the ratio of croscarmellose sodium to sodium stearyl fumarate is in the range of preferably 1 to 10 (w/w) and more preferably 2 to 5 (w/w).

10. The method according to any of the preceding claims; wherein said formulation does not comprise magnesium stearate.

11. The method according to any of the preceding claims; wherein the method is used for preparing the pharmaceutical formulation consisting of;
a) 5 to 60% by weight of lacosamide
b) 0.1 to 10% by weight of croscarmellose sodium
c) 2 to 15% by weight of crospovidone
d) 0.25 to 5% by weight polyvinylpyrrolidone - polyvinyl acetate copolymer
e) 0.01 to 1% by weight colloidal silicon dioxide
f) 10 to 60% by weight microcrystalline cellulose
g) 0.1 to 5% by weight sodium stearyl fumarate
h) 0.4 to 5% by weight coating

12. The method according to any of the preceding claims; wherein the method is used for preparing the pharmaceutical formulation comprising;
a) 40% by weight of lacosamide
b) 5.0 % by weight of croscarmellose sodium
c) 8.0 % by weight of crospovidone
d) 2.0 % by weight polyvinylpyrrolidone - polyvinyl acetate copolymer
e) 0.4 % by weight colloidal silicon dioxide
f) 43.1 % by weight microcrystalline cellulose
g) 1.5 % by weight sodium stearyl fumarate
h) 3.0 % by weight coating

13. The method according to any of the preceding claims; wherein minimum 85% of lacosamide by weight relative to the total weight of said formulation is released within 15 minutes when measured according to USP Apparatus II (Paddle) method in 900mL of 0.1N HCl at 50rpm.

14. The method according to claim 11 or 12, comprising the steps of
a) Sieving lacosamide, 50% of sodium stearyl fumarate, 50% of crospovidone, 50% of colloidal silicon dioxide and all other excipients and mixing the resulting mixture,
b) Passing the mixture through a compactor or pressing into a slug briquette.
c) Powders or slug briquetted tablets are crushed and sieved.
d) adding 50% of sodium stearyl fumarate, 50% of crospovidone, 50% of colloidal silicon dioxide and mixing the resulting mixture,
e) pressing resultant mixture,

15. The method according to claim 11 or 12, wherein the compaction is carried out in a roller compactor and the rolling force is 180 to 190 kN.

## Patentansprüche

1. Verfahren zur Herstellung einer pharmazeutischen Formulierung in Form einer Tablette, umfassend eine therapeutisch wirksame Menge von Lacosamid oder eines pharmazeutisch annehmbaren Salzes, Natriumstearylfumarat und wenigstens einen Hilfsstoff; wobei es sich bei dem Verfahren um Trockengranulation handelt.

2. Verfahren nach Anspruch 1; wobei die Menge von Natriumstearylfumarat in einer Menge von 0,01 bis 10 Gew.-% der gesamten Formulierung vorhanden ist; wobei sie vorzugsweise 0,1 bis 5 Gew.-% der gesamten Formulierung beträgt.

3. Verfahren nach den Ansprüchen 1 und 2; wobei das Verhältnis von Lacosamid zu Natriumstearylfumarat im Bereich von 1 bis 100 (w/w), vorzugsweise 20 bis 30 (w/w) liegt.

4. Verfahren nach Anspruch 1; wobei die Formulierung ferner ein Superzerfallhilfsmittel umfasst.

5. Verfahren nach Anspruch 4; wobei das Superzerfallhilfsmittel aus der Gruppe ausgewählt ist, die Croscarmellose-Natrium, Natriumcarboxymethylstärke, Natriumstärkeglycolat, Sojapolysaccharid, vernetzte Alginsäure, Gellangummi, Xanthangummi, Calciumsilicat oder Ionenaustauschharze oder Mischungen davon umfasst.

6. Verfahren nach Anspruch 5; wobei das Superzerfallhilfsmittel Croscarmellose-Natrium ist.

7. Verfahren nach einem der vorhergehenden Ansprüche; wobei Croscarmellose-Natrium im Bereich von 0,1 bis 10 Gew.-%, vorzugsweise 2 bis 7 Gew.-% der gesamten Formulierung vorhanden ist.

8. Verfahren nach einem der vorhergehenden Ansprüche; wobei das Verhältnis von Lacosamid zu Croscarmellose-Natrium im Bereich von 5 bis 50 (w/w), vorzugsweise 2 bis 10 (w/w) liegt.

9. Verfahren nach einem der vorhergehenden Ansprüche; wobei das Verhältnis von Croscarmellose-Natrium zu Natriumstearylfumarat im Bereich von vorzugsweise 1 bis 10 (w/w) und stärker bevorzugt 2 bis 5 (w/w) liegt.

10. Verfahren nach einem der vorhergehenden Ansprüche; wobei die Formulierung kein Magnesiumstearat umfasst.

11. Verfahren nach einem der vorhergehenden Ansprüche; wobei das Verfahren verwendet wird, um die pharmazeutische Formulierung herzustellen, die aus Folgendem besteht:
a) 5 bis 60 Gew.-% Lacosamid,
b) 0,1 bis 10 Gew.-% Croscarmellose-Natrium,
c) 2 bis 15 Gew.-% Crospovidon,
d) 0,25 bis 5 Gew.-% Polyvinylpyrrolidon-Polyvinylacetat-Copolymer,
e) 0,01 bis 1 Gew.-% kolloidales Siliciumdioxid,
f) 10 bis 60 Gew.-% mikrokristalline Cellulose,
g) 0,1 bis 5 Gew.-% Natriumstearylfumarat,
h) 0,4 bis 5 Gew.-% Beschichtung.

12. Verfahren nach einem der vorhergehenden Ansprüche; wobei das Verfahren verwendet wird, um die pharmazeutische Formulierung herzustellen, die Folgendem umfasst:
a) 40 Gew.-% Lacosamid,
b) 5,0 Gew.-% Croscarmellose-Natrium,
c) 8,0 Gew.-% Crospovidon,
d) 2,0 Gew.-% Polyvinylpyrrolidon-Polyvinylacetat-Copolymer,
e) 0,4 Gew.-% kolloidales Siliciumdioxid,
f) 43,1 Gew.-% mikrokristalline Cellulose,
g) 1,5 Gew.-% Natriumstearylfumarat,
h) 3,0 Gew.-% Beschichtung.

13. Verfahren nach einem der vorhergehenden Ansprüche; wobei mindestens 85 Gew.-% Lacosamid bezogen auf das Gesamtgewicht der Formulierung bei Messung nach dem Verfahren USP Apparatus II (Blattrührer) in 900 mL 0,1 N HCl bei 50 UpM innerhalb von 15 Minuten freigesetzt werden.

14. Verfahren nach Anspruch 11 oder 12, umfassend die folgenden Schritte:
a) Sieben von Lacosamid, 50 % des Natriumstearylfumarats, 50 % des Crospovidons, 50 % des kolloidalen Siliciumdioxids und aller anderen Hilfsstoffe und Mischen der entstandenen Mischung,
b) Leiten der Mischung durch einen Verdichter oder Pressen in einer Brikettpresse,
c) Zermahlen und Sieben der Pulver oder brikettierten Tabletten,
d) Zusetzen von 50 % des Natriumstearylfumarats, 50 % des Crospovidons, 50 % des kolloidalen Siliciumdioxids und Mischen der entstandenen Mischung,
e) Pressen der entstandenen Mischung.

15. Verfahren nach Anspruch 11 oder 12, wobei die Verdichtung in einem Walzenverdichter durchgeführt wird und die Walzkraft 180 bis 190 kN beträgt.

## Revendications

1. Procédé de préparation d'une formulation pharmaceutique sous la forme d'un comprimé, comprenant une quantité thérapeutiquement efficace de lacosamide ou d'un sel pharmaceutiquement acceptable, du fumarate de sodium et de stéaryle et au moins un excipient, le procédé étant une granulation par voie sèche.

2. Procédé selon la revendication 1, dans lequel la quantité de fumarate de sodium et de stéaryle est présente dans une quantité de 0,01 à 10 % en poids de la formulation totale, étant de préférence de 0,1 à 5 % en poids de la formulation totale.

3. Procédé selon l'une des revendications 1 et 2, dans lequel le rapport de lacosamide au fumarate de sodium et de stéaryle se situe dans la plage de 1 à 100 (p/p), de préférence de 20 à 30 (p/p).

4. Procédé selon la revendication 1, dans lequel la formulation comprend en outre un super-désintégrant.

5. Procédé selon la revendication 4, dans lequel ledit super-désintégrant est choisi dans le groupe comprenant la croscarmellose sodique, le carboxyméthyl amidon sodique, le glycolate d'amidon sodique, un polysaccharide de soja, l'acide alginique réticulé, la gomme de gellane, la gomme de xanthane, le silicate de calcium ou les résines échangeuses d'ions ou leurs mélanges.

6. Procédé selon la revendication 5, dans lequel ledit super-désintégrant est la croscarmellose sodique.

7. Procédé selon l'une quelconque des revendications précédentes, dans lequel la croscarmellose sodique se situe dans la plage de 0,1-10 %, de préférence de 2-7 %, en poids de la formulation totale.

8. Procédé selon l'une quelconque des revendications précédentes, dans lequel le rapport du lacosamide à la croscarmellose sodique se situe dans la plage de 5 à 50 (p/p), de préférence de 2 à 10 (p/p).

9. Procédé selon l'une quelconque des revendications précédentes, dans lequel le rapport de la croscarmellose sodique au fumarate de sodium et de stéaryle se situe dans la plage de préférence de 1 à 10 (p/p) et de façon davantage préférée de 2 à 5 (p/p).

10. Procédé selon l'une quelconque des revendications précédentes, dans lequel ladite formulation ne comprend pas de stéarate de magnésium.

11. Procédé selon l'une quelconque des revendications précédentes, dans lequel le procédé est utilisé pour préparer la formulation pharmaceutique consistant en :
a) 5 à 60 % en poids de lacosamide ;
b) 0,1 à 10 % en poids de croscarmellose sodique ;
c) 2 à 15 % en poids de crospovidone ;
d) 0,25 à 5 % en poids de copolymère polyvinylpyrrolidone-poly(acétate de vinyle) ;
e) 0,01 à 1 % en poids de dioxyde de silicium colloïdal ;
f) 10 à 60 % en poids de cellulose microcristalline ;
g) 0,1 à 5 % en poids de fumarate de sodium et de stéaryle ;
h) 0,4 à 5 % en poids d'enrobage.

12. Procédé selon l'une quelconque des revendications précédentes, dans lequel le procédé est utilisé pour préparer la formulation pharmaceutique comprenant :
a) 40 % en poids de lacosamide ;
b) 5,0 % en poids de croscarmellose sodique ;
c) 8,0 % en poids de crospovidone ;
d) 2,0 % en poids de copolymère polyvinylpyrrolidone-poly(acétate de vinyle) ;
e) 0,4 % en poids de dioxyde de silicium colloïdal ;
f) 43,1 % en poids de cellulose microcristalline ;
g) 1,5 % en poids de fumarate de sodium et de stéaryle ;
h) 3,0 % en poids d'enrobage.

13. Procédé selon l'une quelconque des revendications précédentes, dans lequel 85 % au minimum de lacosamide en poids par rapport au poids total de ladite formulation sont libérés en l'espace de 15 minutes lorsqu'ils sont mesurés conformément à la méthode de l'Appareil USP II (Palette) dans 900 mL de HCl 0,1N à 50 tpm.

14. Procédé selon l'une des revendications 11 ou 12, comprenant les étapes de :
a) tamiser le lacosamide, 50 % du fumarate de sodium et de stéaryle, 50 % de la crospovidone, 50 % du dioxyde de silicium colloïdal et tous les autres excipients et mélanger le mélange résultant ;
b) faire passer le mélange à travers un compacteur ou le comprimer en une briquette comprimée ;
c) les poudres ou les comprimés en briquettes comprimées sont broyés et tamisés ;
d) ajouter 50 % du fumarate de sodium et de stéaryle, 50 % de la crospovidone, 50 % du dioxyde de silicium colloïdal et mélanger le mélange résultant ;
e) comprimer le mélange résultant.

15. Procédé selon l'une des revendications 11 ou 12, dans lequel le compactage est effectué dans un compacteur à rouleau et la force de roulage est de 180 à 190 kN.
